**Europäisches Patentamt**

**European Patent Office**

**Office européen des brevets**

(11) Publication number: **0 209 493**
A2

# (12) EUROPEAN PATENT APPLICATION

(21) Application number: 86810304.5

(22) Date of filing: 10.07.86

(51) Int. Cl.⁴: **A 61 K 39/395**
// C12P21/00, C12N5/00,
C12N15/00, A61K9/12

(30) Priority: 16.07.85 US 755645

(43) Date of publication of application: 21.01.87
**Bulletin 87/4**

(84) Designated Contracting States: BE CH DE FR GB IT LI LU NL SE

(71) Applicant: CIBA-GEIGY AG, Klybeckstrasse 141, CH-4002 Basel (CH)

(72) Inventor: Tuomanen, Elaine, Dr., 450 East 63rd Street Apt. 121, New York New York 10021 (US)
Inventor: Rosenfelder, Günter, Dr., Am Sonnenrain 21, D-7851 Binzen (DE)
Inventor: Towbin, Harry, Dr., Feldstrasse 31, CH-4123 Allschwil (CH)
Inventor: Braun, Dietmar, Prof. Dr., Wyhlenweg 4, CH-4126 Bettingen (CH)

(54) Prophylaxis and treatment of whooping cough.

(57) A method is disclosed of preventing and/or treating whooping cough caused by *Bordetella pertussis* by administering monoclonal antibodies raised against mammalian cell membrane glycoconjugates.

EP 0 209 493 A2

4-15382/+/TUO

Prophylaxis and treatment of whooping cough

Field of the invention
The invention relates to a method of preventing and/or treating
whooping cough caused by Bordetella pertussis by administering
monoclonal antibodies raised against mammalian cell membrane
glycoconjugates.

Background of the invention
Bordetella species cause infections which are localized at the
ciliated respiratory mucosa in humans and animals. B. pertussis and
B. parapertussis produce natural disease only in humans, whereas
B. bronchiseptica causes disease predominantly in animals, e.g.
rodents or dogs. Whooping cough (pertussis) caused by Bordetella
pertussis is a disease of considerable incidence even in highly
vaccinated human populations and contributes heavily to infant
mortality in less developed nations.

The World Health Organziation currently recommends that every child
worldwide receives protection from whooping cough. The current
inactivated whole cell vaccine available in the United States and
Europe is 70-90 % effective but has an unacceptably high rate of
toxicity including death (C.V.Broome and D.W.Fraser, J.Inf.Dis. 144,
187 (1981)). New vaccines such as that discussed by V.A.Fulginiti,
J.Inf.Dis. 148, 146 (1983) containing only antigenic fractions of
B. pertussis may eventually reduce toxicity, however, such a vaccine
will not be able to confer 100 % protection to those vaccinated over
a prolonged time period and will not be useful in the therapy of the
disease.

Chemotherapeutics now available for the treatment of whooping cough are of limited value, because they only can ease the progress of the disease, but not really combat the infection. There is a need for more effective treatment. It is the object of the invention to provide such a method.

Description of the invention

Monoclonal antibodies raised against mammalian cell membrane glycoconjugates are highly effective in the prevention and treatment of whooping cough caused by B. pertussis and infections of the respiratory tract caused by other Bordetella species. Accordingly, the invention relates to a method for preventing and/or treating whooping cough caused by Bordetella pertussis and infections of the respiratory tract caused by other Bordetella species in a mammal, characterized in that an effective amount of a monoclonal antibody specific for mammalian cell membrane glycoconjugates or a derivative thereof is administered to said mammal in need of treatment.

Glyconconjugates are bio-molecules containing a carbohydrate function, e.g. glycolipids such as glycosphingolipids, glycoproteins, or proteoglycanes.

Especially useful in this invention are monoclonal antibodies and derivatives thereof which bind to neutral glycosphingolipids produced by epithelial cells as membrane constituents, in particular to neutral glycosphingolipids produced by malignant epithelial cells, such as mammary, lung or colorectal carcinoma cells, especially mammary carcinoma cells, or to neutral glycosphingolipids obtained from meconium. Glycosphingolipids (GSL) consist of a carbohydrate, which is a mono- or usually an oligosaccharide, glycosidically bound to the primary hydroxyl function of a long chain amino-alcohol, the most common being sphingosine, which in turn is linked to a fatty acid via an amide bond. Such glycosphingolipids may be incorporated in the cell membrane of the epithelial cells or may be released by them into the body fluids.

Preferred are the monoclonal antibodies with the designation 1018S19.11, 1018S69.4, 1021S11.28, 1022S25.17 and 1022S23.24, and derivatives thereof, such as antibody fragments, but particularly the monoclonal antibodies themselves. Fragments of monoclonal antibodies of this invention are e.g. Fab, Fab' or $F(ab')_2$ fragments, which retain their specificity for the antigenic determinants, i.e. which retain the characteristic binding pattern of the parent monoclonal antibody to glycosphingolipids.

The monoclonal antibodies of the invention are detected and analyzed with regard to their binding pattern towards glycosphingolipids (GSL) from various sources and characterized by their immunoglobulin class or subclass.

The binding pattern of monoclonal antibodies to GSL can be determined e.g. by the so-called GSL-blotting technique. The glycosphingolipids are obtained by known methods, e.g. by solvent extraction, solvent distribution and chromatographic methods, from various tissues, such as breast, liver, kidney, spleen, tonsil tissue, also tumour tissues, e.g. mammacarcinoma, lung tumours and the like, from erythrocytes and other cellular sources, for instance meconium, i.e. the faeces of newborns. The GSL are separated by thin layer chromatography, and the entire chromatographic pattern transferred to nitrocellulose. To that end, the thin layer plate is moistened with a suitable solvent such as an aqueous lower alcohol and pressed on a sheet of porous nitrocellulose. The obtained replica on nitrocellulose is optionally cut into strips and incubated with solutions containing monoclonal antibodies, and any immunological reaction of the antibodies with the glycosphingolipids adsorbed on the nitrocellulose detected by methods known in the art, e.g. by development with a second antibody conjugated to an enzyme followed by an enzyme substrate or development with a radioactively labelled second antibody.

The monoclonal antibodies of the invention bind to neutral GSL in most of the GSL extracts from breast tumor biopsy material from 19 individual humans, but do not bind to gangliosides, i.e. sialic acid containing GSL. These results together with the approximate number of monosaccharide units of the carbohydrate chain of the antigenic neutral GSL are shown in the Table.

The immunoglobulin class and subclass of the monoclonal antibodies is determined by well-known methods, e.g. the immunodiffusion Ouchterlony technique using class-specific second antibodies. The monoclonal antibodies of the invention belong to the class of the immunoglobulins $G_1$, $G_{2a}$, $G_{2b}$, $G_3$, or M.

The monoclonal antibodies of this invention bind to mammalian ciliated cells, e.g. human or rabbit ciliated cells from trachea. The ability of monoclonal antibodies to bind to ciliated cells is determined in an assay, wherein freshly prepared suspensions of viable ciliated cells are incubated with the monoclonal antibodies. After washing, the monoclonal antibodies bound to the cells are detected by a fluorescein-conjugated second antibody recognizing epitopes on the monoclonal antibodies to be tested. The results of this assay are shown in the Table.

By their ability to bind to ciliated cells, the monoclonal antibodies of this invention block the adherence of B. pertussis to cilia. Presumably this arises because the antibodies bind with and/or render inaccessible the receptor for B. pertussis on the ciliary membrane. The ability of monoclonal antibodies to block the adherence of B. pertussis to ciliated cells is determined in an adherence assay as described by E.I. Tuomanen et al., J. Clin. Micro. 20, 167 (1984). In this assay, viable B. pertussis are mixed with the monoclonal antibodies to be tested and subsequently incubated with freshly prepared suspensions of viable cells, which cells are optionally preincubated with adhesins, e.g. filamentous hemagglutinin or pertussis toxin. B. pertussis adhering to the ciliated cells are detected by a fluorescein-conjugated antibody binding to the bacteria, but not to the cells or to the monoclonal antibodies to be tested. In a parallel assay it is shown that the monoclonal antibodies used in the invention do not bind to B. pertussis. The results are collected in the Table.

In part, the monoclonal antibodies of the invention are able to displace B. pertussis from human ciliated cells. This is determined in a modified adherence assay as described hereinbefore. The results are collected in the Table.

Table: Characterization of monoclonal antibodies

| Antibody designation | 1018S19.11 | 1018S69.4 | 1021S11.28 | 1022S25.17 | 1022S23.24 |
|---|---|---|---|---|---|
| Source of antigen used for immunization (Examples 1 and 2) | - - human mammary carcinoma - - | | | - - human meconium -- | |
| Antibody class (Example 5) | $IgG_1$ | $IgG_3$ | $IgG_{2a}$ | $IgG_{2b}$ | $IgG_{2b}$ |
| Binding to GSL from different mammary biopsies (Example 6) | 11/19* | 17/19 | 11/16 | n.t. | 15/16 |
| Approx. number of monosaccharides in antigenic neutral GSL | 5 | 6 | 5 | 5 | 5 |
| Binding to (Example 9) human ciliated cells rabbit ciliated cells B. pertussis | + + - | + + - | - - - | + + - | - + - |
| Antiadherence activity in (Example 8) human ciliated cells rabbit ciliated cells | + + | + + | - + | ++ ++ | ++ - |
| Displacement of B. pertussis from human ciliated cells (Example 10) | - | n.t. | - | + | + |

*11 positive out of a total of 19
n.t. = not tested

The results of the <u>B. pertussis</u> adherence test on human and rabbit ciliated cells collected in the Table demonstrate that the monoclonal antibodies are applicable to the prevention and/or treatment of whooping cough and related infections of the respiratory tract caused by <u>Bordetella</u> species. Direct delivery of these monoclonal antibodies, or fragments thereof which retain their specificity for the antigenic determinants, to the ciliated respiratory mucosa lead to binding to the <u>B. pertussis</u> receptor, thereby inhibiting the adherence of the bacteria to their specific host target. <u>B. pertussis</u> can then be cleared by the ciliary escalator and infection avoided. Such a prophylaxis is particularly important because the disease is virtually 100 % contagious and significant doubt exists as to the efficacy of any antibiotic therapy in preventing colonization of <u>B. pertussis</u> on the cilia.

Furthermore, efficient therapy of established cases of whooping cough and related diseases is envisioned. The infection, which lasts approximately 6-8 weeks, is perpetuated by multiplication of <u>B. pertussis</u> adherent to the cilia and subsequent attachment of new daughter cells to new receptor sites. Administration of the monoclonal antibodies will block the attachment of such daughter cells and compete with established cells for the binding sites on the cilia, thereby attenuating and finally aborting the infection.

According to the invention, the monoclonal antibodies are administered to the respiratory tract of a mammal including man. They may be delivered in the form of inhalation compositions, e.g. as a powder mixture with a particle size of preferably from about 2 to 5 µm, as an aqueous solution to be sprayed with a nebulizer, or as an aerosol from a pressurized container with the aid of a volatile propellant.

Pharmaceutical compositions in powder form are prepared by mixing the monoclonal antibodies of the invention with a solid carrier compatible with lung tissue, preferably lactose, and optionally stabilisers. The powders are sieved and packaged in a device adapted to emit a measured amount of powder when inhaled, optionally together with a propellant. Suitable propellants are compressed gases, such as compressed air, also laughing gas or carbon dioxide.

Pharmaceutical compositions for inhalation are also aqueous solutions, e.g. isotonic solutions and buffer solutions compatible with lung tissue. These solutions are prepared by dissolving the monoclonal antibodies in water or aqueous salt solutions, optionally adding stabilisers and preservatives as required. The solutions are filled in a spray device or nebulizer suitable for inhalation through the mouth.

Aerosols are prepared by dissolving the monoclonal antibodies in water, adding an alcohol, such as ethanol or benzyl alcohol, and stabilizers as required, and mixing it with a liquefied volatile propellant. The mixture is filled in a pressurized container having a valve releasing a predetermined amount of aerosol. Suitable propellants are non-toxic and have a boiling point below room temperature at atmospheric pressure, and are e.g. lower alkanes or lower alkyl halides. Particularly suitable are lower fluoroalkanes or lower chlorofluoroalkanes known under the trade mark "Freon", e.g. dichlorodifluoromethane (Freon® 12), dichlorotetrafluoroethane (Freon® 114) and the like.

The dosage of the monoclonal antibody to be administered depends on the species of the mammal, the age and the individual condition of the mammal in need of treatment. The daily dose administered to the respiratory tract, for example, of an infant of 10 kg body weight is from approximately 5 mg to approximately 500 mg, preferably from approximately 10 mg to 100 mg. Adults of e.g. 70 kg body weight obtain higher doses, for example three to ten times the doses stated, preferably from approximately 100 mg to 1000 mg.

The monoclonal antibodies used in the invention and derivatives
thereof are obtained by processes known per se, characterized in
that hybridoma cells secreting said monoclonal antibodies
a) are cultivated in vitro and the monoclonal antibodies isolated
   from the culture supernatant, or
b) are propagated in vivo in a suitable mammal and the monoclonal
   antibodies recovered from body fluids of said mammal, and, if
   desired
c) the obtained monoclonal antibodies are converted into a derivative thereof.

Suitable culture media for the in vitro cultivation according to
process a) are standard culture media such as Dulbecco's Modified
Eagle Medium or RPMI 1640 Medium, optionally replenished by a mammal
serum, e.g. fetal calf serum. The isolation of the monoclonal
antibodies is accomplished by precipitating the protein contained in
the culture supernatants by ammonium sulfate or the like, followed
by purifying the immunoglobulins by standard chromatographic
methods, such as gel filtration, ion exchange chromatography,
chromatography on DEAE cellulose, or immunoaffinity chromatography.

Large amounts of the desired monoclonal antibodies can be obtained
by the propagation of hybridoma cells according to process b). Cell
clones are injected into syngeneic mammals, which causes antibody-
producing tumours to grow. After one to three weeks the desired
monoclonal antibodies are recovered from body fluids of said mammal.
As an example hybridoma cells derived from Balb/c mice are intraperitoneally injected into Balb/c mice optionally pretreated with a
hydrocarbon such as pristane, and after one to two weeks, ascites
fluid of these mice is collected. The desired monoclonal antibodies
are isolated from the body fluids by methods known per se, e.g. by
precipitating the proteins with ammonium sulfate or the like,
followed by purifying the immunoglobulins by standard chromatographic methods, such as gel filtration, ion exchange chromatography, chromatography on DEAE cellulose, or immunoaffinity chromatography.

Fragments of monoclonal antibodies, for example Fab, Fab' or F(ab')$_2$ fragments, which retain their specificity towards the antigenic determinants of the glycosphingolipids, can be obtained from the monoclonal antibodies prepared according to process a) or b) by methods known per se, e.g. by digestion with enzymes such as pepsin or papain and/or cleavage of disulfide bonds by chemical reduction.

Examples of hybridoma cell lines secreting the desired antibodies are cell lines, which are hybrids of myeloma cells and B lymphocytes of a mammal immunized with glycosphingolipids. Preferentially, these cell lines are hybrids of mouse myeloma cells and B lymphocytes of a syngeneic mouse immunized with glycosphingolipids.

Particularly preferred are the monoclonal antibodies secreted by the hybridoma cell lines with the designation 1018S19.11, 1022S25.17, 1021S11.28, and 1018S69.4, which have been deposited on August 16, 1984 under the number I-331, I-332, I-333, and I-334, respectively, and with the designation 1022S23.24, which has been deposited on May 31, 1985 under the number I-453, with the "Collection Nationale de Cultures de Microorganismes", Institut Pasteur, Paris. These hybridoma cell lines are hybrids of the mouse myeloma cell line Sp2/0-Ag14 and of B lymphocytes of the spleen of Balb/c mice immunized with glycosphingolipids. They are stable cell lines, which secrete the monoclonal antibodies with the designation 1018S19.11, 1022S25.17, 1021S11.28, 1018S69.4, and 1022S23.24, respectively. The cell lines may be kept in deep-frozen cultures and reactivated by thawing and re-cloning.

The mentioned preferred hybridoma cell lines secreting monoclonal antibodies to neutral glycosphingolipids are prepared by immunizing Balb/c mice with glycosphingolipids adsorbed on Salmonella minnesota R595 rough mutant bacteria, fusing antibody-producing spleen cells of these mice with syngeneic myeloma cells, cloning the hybrid cells obtained in the fusion, and selecting cell clones secreting the desired antibodies.

The glycosphingolipids used for the immunization are obtained from
human mammary carcinoma biopsy material pretreated with acetone or
from human meconium by extraction with methanol/chloroform/water-
mixtures, solvent partition between water and methanol/chloroform,
and chromatography over reversed phase silica gel.

The following examples illustrate the invention, but do not limit it
to any extent.

The abbreviations used in the examples have the following meaning:

BSA     bovine serum albumin
GSL     glycosphingolipids
HAT     hypoxanthine/aminopterin/thymidine
HEPES   N-2-hydroxyethylpiperazine-N'-2-ethanesulfonic acid
HPTLC   high performance thin layer chromatography
PBS     phosphate buffered saline
TBS     tris-(hydroxymethyl)-aminomethane buffered saline
Tris    tris-(hydroxymethyl)-aminomethane
VCB     viral collecting broth

Example 1: Isolation and purification of polar glycosphingolipids
           from mammary carcinoma biopsy material.

1.1 Extraction: Cellular sediments derived from carcinoma biopsy
tissue specimen taken for estrogen receptor determinations are
obtained from a large number of patients with histologically
confirmed diagnosis of mammary carcinoma. In a typical experiment,
190 individual samples obtained as frozen clots in polystyrene tubes
are further cooled down in dry ice and the plastic is smashed with a
hammer. The collected sediments (169 g wet weight) are lyophilized
and yield 33 g dry weight. The dry material is freed from storage
fat by repeated homogenization in acetone at 4°C using a Sorval
tissue blender and subsequent filtration. The residual solid
material still contains the bulk of polar lipids such as phospholipids and glycosphingolipids. These are extracted at room temperature three times using 200 ml of chloroform/methanol 2:1 (v/v) and twice with 200 ml chloroform/methanol/water 1:1:0.2 (v/v/v).

1.2 Folch partition: The bulk of phospholipids and those glycosphingolipids having less than four sugars in their oligosaccharide chain are separated from polar glycosphingolipids by a modified partition procedure according to Folch (T. Saito and S. Hakomori, J. Lipid Res. 12, 257 (1971)). The collected extracts from Example 1.1 are evaporated under reduced pressure at 40°C and redissolved in 90 ml of chloroform/methanol 2:1 (v/v) in a stoppered glass cylinder. 15 ml of water are added and the solutions are vigorously shaken. After 15 min standing the phases have separated. The lower chloroform-rich phase is extracted three times with chloroform/methanol/0,1 % aqueous KCl 1:10:10 (v/v/v).

1.3 Reversed phase chromatography: To remove salt and other watersoluble contaminants, the combined upper phases from Example 1.2 are evaporated, taken up in water and applied to a glass column containing 7 ml of Lichroprep® RP18 (Merck, F.R.G.). After extensive washing with water the polar glycosphingolipid fraction is eluted with 10 ml of methanol and then 10 ml of chloroform/methanol 2:1 (v/v). The yield is about 30 mg, i.e. 0,1 % of tissue dry weight.

1.4 Thin layer chromatography: The purity of the GSL preparation is tested by silica gel thin layer chromatography and subsequent spraying of the plates with either orcinol/sulphuric acid reagent visualizing sugar containing compounds (L. Svennerholm, J. Neurochem. 1, 42 (1956)) or Phospray® (Supelco Inc., Bellefonte, Pa., USA, see J.C. Dittmer and R.L. Lester, J. Lipid Res. 5, 126 (1964)) to detect phospholipids. Silica gel 60 HPTLC plates (size 5x5 cm, thickness of sorbent layer 0.24 mm, Merck, F.R.G.) are used. The lipid samples are dissolved in 10-20 µl of chloroform/methanol 2:1 (v/v) and applied to the plates as a streak of 4 cm length using an automated sample applicator model Linomat III (Camag, Switzerland). The solvent system is chloroform/methanol/ water 55:45:10 (v/v/v) containing 1 mM CaCl$_2$. The developed plates show the typical pattern of GSL mixtures with carbohydrate residues of varying monosaccharide content. Only traces of non-glycosphingolipid material are present.

Example 2: <u>Isolation and purification of glycosphingolipids from meconium</u>

Newborn feces from various individuals are scraped from diapers and treated exactly the same way as described in Example 1 with the exception that the acetone-extraction step of Example 1.1 is omitted. The yield is about 0.2 % of dry weight. The composition and purity of the glycosphingolipid (GSL) mixture is determined as described in Example 1.4.

Example 3: <u>Production of hybridoma cells</u>

3.1 <u>Preparation of immunogen</u>: Purified GSL from Example 1 or 2 are adsorbed to freeze dried and acetic acid-treated <u>Salmonella</u> <u>minnesota</u> R-595 bacteria (C. Galanos, O. Lüderitz and O. Westphal, Eur. J. Biochem. <u>24</u>, 116 (1971)) to increase the immunogenicity of lipid antigens. To that end, 50 mg of bacteria (dry weight) are suspended in a 250 ml round bottom flask in 100 ml of chloroform/methanol 2:1 (v/v) containing 10 mg of GSL. The solvent is evaporated at room temperatrure using a rotary evaporator revolving at full speed. GSL covered bacteria are scraped from the glass wall and suspended in phosphate buffered saline (PBS) at a concentration of 2.4 mg/ml. Upon microscopical evaluation irregularly shaped clumps as well as single bacteria are observed.

3.2 <u>Immunization protocol</u>: 4 female Balb/c mice are injected intraperitoneally each with samples of GSL-covered bacteria (2 with GSL from mammary carcinoma, 2 with GSL from meconium) containing 100 μg of GSL on day 0, 200 μg on day 4, 300 μg on day 7, and 400 μg on day 11. On day 19 the mice are bled orbitally and tested for anti-GSL serum activity using the GSL-blotting system described in Example 6. On day 36 a booster injection of a sample containing 400 µg GSL is given and the mice are sacrificed four days latter.

3.3 Cell fusion: All fusion experiments are performed according to the procedure of G. Köhler and C. Milstein (Nature 256, 495 (1975)) using the nonsecreting Sp 2/0-Ag14 myeloma line (M. Shulman, C.D. Wilde and G. Köhler, Nature 276, 269 (1978)). $10^8$ spleen cells are mixed with $10^7$ myeloma cells in the presence of 1 ml of 50 % polyethylene glycol (PEG 1500, Serva). After washing, the cells are resuspended in 48 ml of standard Dulbecco's minimum essential medium (Gibco No. 0422501). 15 % fetal calf serum and $3x10^6$ normal mouse peritoneal exsudate cells per fusion are added as feeder cells. The cells are distributed into 48 x 1 ml costar wells and fed 3 to 4 times per week with standard HAT selection medium for 3 to 6 weeks. When the growth of hybridoma cells becomes visible, the supernatants are tested for binding to GSL (Example 6). Cloning of hybridoma cells is done by limiting dilution in microtiter plates. The supernatants are tested for binding to GSL in a solid phase enzyme immunoassay described in Example 7. All hybridoma lines are cloned at least once. From a total of 12 hybridoma cell lines positive in the ELISA of Example 7, the clones with the designation 1018S19.11, 1018S69.4, 1021S11.28, 1022S25.17, and 1022S23.24 are selected for further characterization.

Example 4: Isolation and purification of monoclonal antibodies
Balb/c mice 8-10 weeks of age (Tierfarm Sisseln, Switzerland) are pretreated intraperitoneally with 0.3 ml pristane (Aldrich). 2-3 weeks later, $2-5x10^6$ cloned hybridoma cells and 0.2 ml pristane are inoculated intraperitoneally. After 8-10 days ascites fluid is collected, centrifuged at 800 x g and stored at -20°C.

Defrosted ascites fluid is centrifuged at 50000 x g for 60 min. A fat layer floating on the surface is carefully removed, and the protein concentration is adjusted to a concentration of 10-12 mg/ml. Crude immunoglobulin is precipitated by dropwise addition of 0.9 volume equivalents of saturated ammonium sulphate at 0°C, then dissolved in 20 mM Tris-HCl/50 mM NaCl (pH 7.9) and dialysed against the same buffer. An immunoglobulin G fraction is obtained by DEAE-D52 cellulose (Whatman) chromatography using a buffer gradient system of 20 mM Tris-HCl/25-400 mM NaCl, pH 7.9. The immunoglobulin G is again precipitated with ammonium sulphate and dissolved in PBS at a concentration of 10 mg/ml. If desired, the immunoglobulin solution is lyophilized and stored in solid form.

Sodium dodecyl sulphate polyacryl amide gel electrophoresis (SDS-PAGE) demonstrates a purity grade of more than 95 percent for the monoclonal antibodies 1018S19.11, 1018S69.4, 1021S11.28, 1022S25.17, and 1022S23.24.

Example 5: Determination of class and subclass of monoclonal antibodies.

The class and subclass of monoclonal antibodies produced by cloned hybridoma cells is determined in an enzyme immunoassay. Microtiter plates are coated with 1 μg per well of a rabbit immunoglobulin preparation of a class- or subclass-specific serum in 50 μl of PBS. Free binding capacity of the plate is saturated with a buffer of 1 % bovine serum albumin in PBS containing 0.2 % $NaN_3$ (w/v), pH 7.4. 100 μl probes containing monoclonal antibodies are incubated in the wells at 37°C for 1 h. The plates are washed with PBS, then incubated at 37°C for 1 h with a phosphatase conjugated rabbit immunoglobulin preparation of the specificity as used for coating the plates. The fixed enzyme is developed by incubating (37°C, 30 min) with a solution of the enzyme substrate p-nitrophenyl phosphate (1 mg/ml in diethanolamine buffer 10 % containing 0.5 mM $MgCl_2$ and 0.02 % (w/v) $NaN_3$, pH 9.8) and measuring the optical density at 405 nm.

The results are collected in the Table.

Example 6: Characterization of the specificity of monoclonal
antibodies against GSL by GSL-blotting

GSL extracts from breast tumor biopsy material are prepared and
separated by thin layer chromatography on HPTLC plates as described
in Example 1. A replica is prepared from the HPTLC plate by transferring the entire pattern of separated GSL to nitrocellulose. This
GSL-blotting assay is especially convenient for screening purposes.

Specifically, 100 µg of human breast biopsy GSL prepared according
to Example 1 are applied as a 10 cm long streak to a HPTLC plate
size 5x10 cm. After separation in the solvent system of Example 1.4,
the plate is dried. Using a soft pencil, lines are drawn as markers
for later orientation. The plate is sprayed with a solution of
iso-propanol/water (2:1) until it is just visibly wet without
formation of droplets on the surface. The plate is immediately
pressed for 1 min on a slightly larger nitrocellulose sheet
(HAWP000®, Millipore) supported by a sheet of polyethylene. Excess
nitrocellulose is cut off and the replica is allowed to dry. The
sheet is rehydrated in a buffer containing 20 mM Tris-HCl/150 mM
NaCl/5 mM $MgCl_2$/0.15 mM $CaCl_2$, pH 7.4 (TBS). The sheet is then
incubated with 10 % horse serum in TBS for 1 h at 40°C to block
excess binding capacity.

The sheet is cut into strips of 2 mm width, each carrying the same
pattern of separated GSL. The strips are incubated for 2 h e.g. with
hybridoma supernatants from Example 3.3 in multipipet reservoir
inserts (Dynatech Laboratories Inc., Alexandria, Va., USA) serving
as suitable incubation trays. The strips are washed three times for
5 min with TBS and incubated for a further 1 h with a conjugate of
horse radish peroxidase and sheep immunoglobulin directed against
mouse IgG (Cappel Laboratories Inc., Chochranville, USA) diluted
500-fold with 10 % (v/v) horse serum in TBS. After washing in TBS as
above, the bound conjugate is visualized by the peroxidase catalyzed
oxidation of 4-chloro-1-naphthol to a blue insoluble deposit. The
substrate 4-chloro-1-naphthol is diluted 50-fold in TBS from a
1 % (w/v) solution in dimethyl formamide, and hydrogen peroxide is added to a final concentration of 0.006 % (w/v). After 20 min the reaction is stopped by washing with water. In the case of a positive reaction one or several bands are seen. Different staining patterns suggest the occurrence of different antibodies.

For the GSL-blotting assay used in the characterization of individual antibodies, 5 µg of human breast biopsy GSL from tissue specimens of 19 individual patients are applied to HPTLC plates and processed as described above. The results of these experiments are collected in the Table.

Example 7: Screening for antibodies against GSL by solid phase enzyme linked immunosorbent assay (ELISA)

A 96-well polyvinylchloride microtiter plate is coated with 200 ng per well of a GSL extract from breast carcinoma biopsy material prepared as in Example 1 dissolved in 25 µl ethanol. The wells are allowed to dry and are completely filled with 1 % (v/v) horse serum in TBS (composition of TBS cf. example 6). After 1 h the plate is washed with TBS, and 50 µl of a solution to be tested for monoclonal antibodies is added. After 3 h the plate is washed and 50 µl of a reagent containing an enzyme-linked second antibody are added. This reagent consists of alkaline phosphatase conjugated goat antibodies against mouse immunoglobulins in a 1000-fold dilution in 1 % (v/v) horse serum in TBS. After 1 h the plate is washed 4 times with TBS and developed with the enzyme substrate p-nitrophenyl phosphate in 10 % diethanolamine buffer as described in Example 5.

**Example 8:** **Bordetella pertussis adherence assay to ciliated cells**

8.1 **Culturing of Bordetella pertussis:** B. pertussis phase I, for example strain Br or BP 338, is isolated and cultured as described by E. Tuomanen et al., J. Clin. Micro. 20, 167 (1984), A. Weiss et al., J. Bacteriology 153, 304 (1983), and A. Weiss et al., Infection and Immunity, 42, 33 (1983). In brief, the organism is cultured on Bordet-Gengou agar or in Stainer-Scholte medium, transferred into medium 199 S (medium 199 supplemented with 0.3 % BSA, 25 mM HEPES, 10 mM L-glutamine, and 0.15 % $NaHCO_3$, M. A. Bioproducts, Walkersville, Md., USA), the bacteria suspension passed three times through a 21-gauge needle to disperse aggregates, and adjusted to the desired concentration of $10^9$ to $10^{10}$ organisms per ml.

8.2 **Preparation of ciliated cells suspension:** Human ciliated cells are obtained by brushing trachea that appear to be normal during bronchoscopy. The cells are suspended in medium 199S to a concentration of 1 to 5 x $10^5$ cells per ml and used in the assay procedure within 12 h of collection. Most cells in the resulting suspension are single; 90 % are ciliated.

Rabbit ciliated cells are obtained by excising tracheas sterilely under pentobarbital anesthesis and scraping cells into medium 199 S with an ear curette. One trachea yields 7-8 ml of a suspension containing 2 x $10^5$ cells per ml.

8.3 **Assay procedure:** 100 µl of the monoclonal antibodies of the invention or of an irrelevant antibody (1 mg/ml) are added to 0.5 ml of B. pertussis suspension (containing $10^{10}$ bacteria per rabbit cell assay and $10^{10}$ or $10^9$ bacteria per human cell assay, the number of organisms being estimated using a Petroff-Hauser counting chamber), incubated for 30 min at 37°C on a shaker, passed three times through a 21-gauge needle, then added to 0.5 ml of a rabbit or human ciliated cell suspension ($10^5$ cells). The mixture is incubated for 3 h at 37°C on a shaker. The cells are rinsed free of nonadherent bacteria over a 3 µm polycarbonate membrane filter

(Nucleopore Corp., Pleasanton, Calif., USA) using 30 - 50 ml of a
1 : 3 mixture of medium 199S and VCB (viral collecting broth:
beef-heart infusion broth with 1 % BSA, Difco), resuspended in
5 ml 199S/VCB 1:3, centrifuged at 250 x g for 15 min, resuspended in
0.1 ml 199S/VCB 1:3, spread onto a microscope slide and air dried
overnight.

The cells are fixed on the slides with acetone, then B. pertussis
organisms that are adherent to ciliated cells stained by adding
10 µl of 1 : 40 dilution of a fluorescent antibody specific for
B. pertussis (Difco Labs., Detroit, Mich., USA). The slides are kept
in a humified chamber for 30 min at 37°, then washed once with
saline and once with saline containing 4 drops 1 % Evans blue as
counterstain. The stained slides are coded and read in a blinded
fashion using a microscope equipped with phase-contrast illumination
and an epifluorescence unit. The mean number of adherent bacteria
per ciliated cell is calculated by examining 25 ciliated cells per
slide.

Without antibodies or in the presence of irrelevant antibodies, a
mean number of five adhering B. pertussis per ciliated cell is found
in preparations containing $10^{10}$ bacteria and $10^5$ human or rabbit
ciliated cells. "Strongly positive" (++) antibodies completely
suppress the adherence (no adhering B. pertussis), whereas
"positive" (+) antibodies give more than zero, but less than two
adhering B. pertussis per ciliated cell in three experiments with
human and with rabbit ciliated cells. The results collected in the
Table are based on the following figures:

| Antibody designation | Number of B. pertussis per ciliary tuft[a] | |
| --- | --- | --- |
| | human | rabbit |
| none (control) | 5 | 5 |
| 1018S19.11 | 1.6 | 1.8 |
| 1018S69.4 | 1.8 | 0.3 |
| 1021S11.28 | 4.3 | 0.8 |
| 1022S25.17 | 0 | 0 |
| 1022S23.24 | 0 | 3.5 |

[a] mean of 3 experiments, results of single experiments within ± 0.5

8.4 Assay in the presence of adhesins: The assay is repeated in the presence of two adhesins, filamentous hemagglutinin and pertussis toxin, known to be responsible for the adherence of B. pertussis to ciliated cells [E. Tuomanen and A. Weiss, J. Infect. Diseases 152, 118 (1985)]. The ciliated cell suspension (Example 8.2) is pre-incubated with 5 µg/ml of filamentous hemagglutinin or pertussis toxin for 30 min, then treated with the mixture of monoclonal antibody and B. pertussis as described in Example 8.3.

Without antibodies or in the presence of irrelevant antibodies, more than ten adhering B. pertussis per ciliated cell are now found in the presence of either adhesin. The following results are obtained with the monoclonal antibodies of the invention:

| Antibody designation | Number of _B. pertussis_ per ciliary tuft | | | |
| --- | --- | --- | --- | --- |
| | human | | rabbit | |
| | + FHA[a] | + TOX[b] | + FHA[a] | + TOX[b] |
| none (control) | > 10 | > 10 | > 10 | > 10 |
| 1018S19.11 | > 10 | > 10 | > 10 | > 10 |
| 1021S11.28 | > 10 | > 10 | 0 | 0 |
| 1022S25.17 | 0 | 0 | 0 | 0 |
| 1022S23.24 | 0 | 7.3 | > 10 | > 10 |

[a] after preincubation with filamentous hemagglutinin

[b] after preincubation with pertussis toxin

The results obtained in the presence of adhesins confirm the general trend of the results obtained without adhesins. Monoclonal antibody 1022S25.17 is generally effective with human and rabbit ciliated cells. Monoclonal antibody 1021S11.28 blocks the adherence of _B. pertussis_ to rabbit ciliated cells only. Monoclonal antibody 1022S23.24 is effective in blocking adherence to human ciliated cells except in the presence of excess pertussis toxin.

Example 9: Binding of monoclonal antibodies to B. pertussis and to ciliated cells

0.5 ml of a suspension of $10^8$ _B. pertussis_ (Example 8.1) or of $10^5$ ciliated rabbit or human cells (Example 8.2) are incubated with 100 µl of the monoclonal antibodies of the invention or of an irrelevant antibody (1 mg/ml) for 30 min at 37°C. The suspension is centrifuged (bacteria: 3000 x g, cells: 250 x g) for 10 min at 4°C, washed twice in 15 ml of medium 199S by resuspending and centrifugation, then spotted on a microscope slide and air dried. The bacteria or cells are fixed on the slides with acetone, then bound monoclonal antibodies detected by staining with 10 µl of a 1 : 20 dilution of the appropriate fluorescein-conjugated anti-mouse immunoglobulin G or M second antibody (Cappell Labs, Malvern, Pa., USA) as described in Example 8.3. The results are shown in the Table.

Example 10: <u>Displacement of B. pertussis bound to human ciliated</u>
cells by monoclonal antibodies

<u>B. pertussis</u> are bound to human ciliated cells in the standard assay
of Example 8.3 omitting monoclonal antibodies. In place of spreading
the cells onto a microscope slide, they are resuspended in 0.5 ml
medium 199S and incubated with 100 µl of the monoclonal antibodies
of the invention (1 mg/ml) for 30 min at 37°C. The cells are rinsed
free of nonadherent bacteria and the standard procedure followed as
described. The results collected in the Table are based on the
following figures:

| Antibody designation | Number of B. pertussis per human ciliated cell remaining adherent[a] |
|---|---|
| none (control) | 5.3 |
| 1018S19.11 | 5.4 |
| 1021S11.28 | 5.2 |
| 1022S25.17 | 2.6 |
| 1022S23.24 | 2.1 |

[a] $p < 0.01$

Monoclonal antibodies 1022S25.17 and 1022S23.14 are capable of
reversing the adherence of bound <u>B. pertussis</u> to human ciliated
cells.

Example 11: Inhalation solution

The monoclonal antibody 1022S25.17 prepared according to Example 4
is dissolved in a solution of a stabiliser and a preservative to
give a inhalation solution of the following composition:

| | |
|---|---|
| monoclonal antibody 1022S25.17 | 2000 mg |
| disodium ethylenediaminetetraacetate | 10 mg |
| benzalkonium chloride | 10 mg |
| water, bidistilled | 100 ml |

The solution is filled in nebulizers suitable for inhalation.

Example 12: Insufflation powder

2000 mg of the monoclonal antibody 1022S25.17 prepared according to
Example 4 are intimately mixed with 10 g finely ground lactose,
then sieved to eliminate particles larger than 5 μm. The powder is
filled into pressure-tight containers with a suitable valve together
with compressed air.

Example 13: Aerosol

500 mg of the monoclonal antibody 1022S25.17 prepared according to
Example 4 are intimately dispersed in 2 ml bidistilled water and
0.5 ml ethanol. The suspension is filled into a pressure-tight
container with a suitable valve together with 5.0 g Freon® 12.

Claims

1. The use of the monoclonal antibodies specific for mammalian cell membrane glycoconjugates or the derivatives thereof in the manufacture of pharmaceutical preparations for preventing and/or treating whooping cough caused by Bordetella pertussis and infections of the respiratory tract caused by other Bordetalla species in a mammal.

2. The use of the monoclonal antibodies according to claim 1 in the manufacture of pharmaceutical preparations for preventing and/or treating whooping cough caused by Bordetella pertussis in a mammal.

3. The use according to claim 1 or 2 of the monoclonal antibodies with the designation 1018S19.11, 1018S69.4, 1021S11.28, 1022S25.17, or 1022S23.24.

4. The use according to claim 1 or 2 of the monoclonal antibody with the designation 1022S25.17.

5. The use according to claim 1 or 2 of the monoclonal antibody with the designation 1022S23.24.

FO 7.4/KB/ga*